# EUROPEAN PATENT APPLICATION

(11) **EP 4 806 360 A1**
(43) Date of publication of application: **16.09.2026**
(21) Application number: 24887977.7
(22) Date of filing: 06.11.2024
(51) Int. Cl.: A61B 6/03

(54) **MEDICAL DEVICE RACK AND MEDICAL DEVICE**

(30) Priority: 06.11.2023 CN 202322999884 U; 27.12.2023 CN 202311836583
(71) Applicant: Shanghai United Imaging Healthcare Co., Ltd., Shanghai 201807 (CN)
(72) Inventor: CHEN, Lisong, Shanghai 201807 (CN); FANG, Li, Shanghai 201807 (CN); MIAO, Junjie, Shanghai 201807 (CN)
(74) Representative: Murgitroyd & Company
(86) International application number: PCT/CN2024/130106
(87) International publication number: WO 2025/098366

(57) **Abstract**

Provided is a gantry for medical device, comprising a fixed assembly (100), a rotating member (200), and a bearing (300). The fixed assembly (100) comprises a first base member (110) and a second base member (120) which are detachably connected. The bearing (300) is mounted on the first base member (110). The rotating member (200) is detachably connected to a rotor (301) of the bearing (300). The second base member (120) is used for support the rotating member (200) separated from the bearing (300).

## Description

### CROSS-REFERENCE TO RELATED APPLICATIONS

This application claims priority of Chinese Patent Application No.202322999884.3, entitled "A Gantry for Medical Imaging Device and Medical Imaging Device", filed on November 6, 2023, and Chinese Patent Application No. 202311836583.7, entitled "A CT Gantry, CT Device, and Heat Dissipation Method for CT Gantry", filed on December 27, 2023, the contents of each of which are entirely incorporated herein by reference.

### TECHNICAL FIELD

The present disclosure relates to the field of medical imaging technology, and in particular, to a gantry for medical device and medical device.

### BACKGROUND

Computed Tomography (CT) uses X-rays to scan specific parts of the human body with a certain thickness. Due to the different absorption ability of different human tissues to X-rays, the image of the section can be reconstructed by computer.

A CT gantry generally comprises a fixed assembly, a rotating member, and a bearing assembly. The rotating member is mounted on the fixed assembly via the bearing assembly. The rotating member carries an X-ray source for emitting an X-ray beam and a detector. After passing through a region of a patient's body, the X-ray beam is captured by the detector. A computer then reconstructs an image of that specific region based on the signals acquired by the detector.

### SUMMARY

An aspect of the present disclosure may provide a gantry for medical device. The gantry comprises a fixed assembly, a rotating member, and a bearing. The fixed assembly comprises a first base member and a second base member which are detachably connected. The bearing is mounted on the first base member. The rotating member is detachably connected to a rotor of the bearing. The second base member is configured to support the rotating member after the rotating member is separated from the bearing.

In some embodiments, the gantry for medical device also comprises a connector, the connector is configured to secure the rotating member to the second base member, and the connector is detachably connected to the rotating member.

In some embodiments, a count of the connector at is at least two, and the at least two of the connectors are configured to connect the rotating member to the second base membe at different positions on the rotating member.

In some embodiments, a guidance structure is provided between the first base member and the second base member, and the guidance structure is configured to guide the first base member and the second base member in an axial direction of the bearing during assembly.

In some embodiments, wherein the gantry for the medical device comprises two rotating members, the two rotating members includes a first rotating member and a second rotating member, each of the first rotating member is connected to the bearing respectively; wherein the second base member is configured to supporting the first rotating part after the first rotating member is detached from the bearing , and wherein the second rotating member is mounted on the first base member through the bearing after the first rotating member is detached from the bearing.

In some embodiments, a synchronizer is disposed on the first base member, the synchronizer being connected to the rotor of the bearing, and the synchronizer having opposite ends connected to the first rotating member and the second rotating member, respectively.

In some embodiments, the first base member has a first channel, and the rotating member has a second channel, the second channel being in communication with the first channel; and wherein an airflow generating assembly is disposed on the first base member, with an air inlet of the airflow generating assembly is in communication with the first channel and an air outlet of the airflow generating assembly is in communication with the second channel.

In some embodiments, a cooler is provided within the first channel and the cooler is configured to perform heat exchange with a gas flowing out of the first channel.

In some embodiments, the first base member has a first end face and the rotating member has a second end face, with the first end face and the second end face are opposite and spaced apart from each other; a protruding ring extends from a periphery of the second end face towards the first end face, such that the first end face, the second end face, and the protruding ring together define an isolation chamber therebetween, and the isolation chamber is in communication between the first channel and the second channel.

In some embodiments, the gantry for the medical device includes a computed tomography (CT) gantry.

Another aspect of the present disclosure may provide a medical device, comprising the gantry for the medical device of any one of the foregoing embodiments.

Another aspect of the present disclosure may provide a gantry for medical device, comprising: a fixed gantry, internally provided with a first channel; a rotatinggantry, rotatably connected to the fixed gantry, wherein the rotatinggantry is internally provided with a second channel; an airflow generating assembly, connected to the fixed gantry, wherein an air inlet of the airflow generating assembly is in communication with the fixed gantry, and an air outlet of the airflow generating assembly is in communication with the second channel; and wherein the fixed gantry, the airflow generating assembly, and the second channel collectively define a passage for circulating gas.

In some embodiments, the air inlet of the airflow generating assembly faces the first channel, and the air outlet of the airflow generating assembly faces the rotatinggantry.

In some embodiments, the gantry for medical device comprises a cooler connected between the fixed gantry and the airflow generating assembly; wherein the cooler is configured to perform heat exchange with gas flowing out of the first channel .

In some embodiments, the cooler is installed within the first channel; the cooler comprises a gas channel and a fluid channel, the gas channel is in communication with the first channel, and the fluid channel is configured to allow a coolant to flow so as to perform heat exchange with gas in the gas channel.

In some embodiments, the gas channel comprises a plurality of ventilation holes arranged spaced apart along a circumference of the cooler, and wherein each of the plurality of ventilation holes is in communication with the first channel.

In some embodiments, the gantry for the medical device comprises a connector assembly movably connected to the fixed gantry, wherein: the connector assembly comprises a connecting pipe and a rotary joint connected to the connecting pipe; the connecting pipe is configured to be connected to a coolant unit; and the rotary joint is configured to be in communication with the fluid channel of the cooler so as to introduce a coolant into the the fluid channel or discharge the coolant from the fluid channel.

In some embodiments, a sliding slot is provided on the fixed gantry, and the connector assembly is configured to be operably slidable within the sliding slot.

In some embodiments, the first channel comprises at least one segmental channel with at least one said segmental channel, the at least one segmental channel is arranged along a circumferential direction and/or a radial direction of the fixed gantry, and/or wherein the second channel comprises at least one annular channel, the at least one annular channel is arranged along a circumferential direction and/or a radial direction of the rotatinggantry, or wherein the second channel comprises at least one segmental channel, the at least one segmental channel is arranged along a circumferential direction and/or a radial direction of the rotating gantry.

Another aspect of the present disclosure may provide a medical device, comprising the gantry for the medical device according to any one of the foregoing embodiments.

Details of the various embodiments of the present invention are set forth in the following description and accompanying drawings. Other features, problems addressed, and technical effects of the present invention will be readily understood by those skilled in the art from the description, the drawings, and the claims.

### BRIEF DESCRIPTION OF THE DRAWINGS

In order to more clearly illustrate the technical solutions in the embodiments of the present application or the prior art, the following will briefly introduce the drawings that need to be used in the description of the embodiments or the prior art. It is obvious that the drawings in the following description are only some embodiments of the present application. For those of ordinary skill in the art, other drawings can be obtained based on these drawings without any creative effort.
FIG. 1 is a schematic diagram illustrating exemplary the three-dimensional structure of the CT gantry provided in an embodiment of this application;
FIG. 2 is a schematic diagram illustrating exemplary partial exploded view of a CT gantry in an embodiment of this application;
FIG. 3 is a schematic diagram illustrating exemplary exploded structure of a CT gantry in an embodiment of this application;
FIG. 4 is a schematic diagram illustrating exemplary perspective view of a CT gantry in another embodiment of this application;
FIG. 5 is a schematic diagram illustrating exemplary partial exploded view of a CT gantry in another embodiment of this application;
FIG. 6 is a schematic diagram illustrating exemplary partial cross-sectional view of a CT gantry in another embodiment of this application;
FIG. 7 is a schematic diagram illustrating exemplary the three-dimensional structure of a gantry provided in an embodiment of this application;
FIG. 8 is a schematic diagram illustrating partial exploded view of the gantry shown in FIG. 7;
FIG. 9 is a schematic diagram illustrating cross-sectional view of the gantry shown in FIG. 7;
FIG. 10 is a schematic diagram illustrating the cooler and the airflow generating assembly in the gantry shown in FIG. 7.
FIG. 11 is a schematic diagram illustrating exemplary a cooler and an airflow generating assembly in a gantry provided in an embodiment of this application;
FIG. 12 is a schematic diagram illustrating the connector assembly in the gantry shown in FIG. 7.
FIG. 13 is a schematic diagram illustrating a connector assembly in a gantry provided in an embodiment of this application.

### DETAILED DESCRIPTION

To make the technical problems to be solved, technical solutions and beneficial effects of the present application clearer and more understandable, the present application will be described in further detail below in combination with the accompanying drawings and embodiments. It should be understood that the specific embodiments described herein are only used to explain the present application, and are not intended to limit the present application.

It should be noted that when an element is referred to as being "fixed on" or "arranged on" another element, it may be directly arranged on the other element or indirectly arranged on the other element. When an element is referred to as being "connected to" another element, it may be directly connected to the other element or indirectly connected to the other element.

It should be understood that the terms such as length, width, upper, lower, front, rear, left, right, vertical, horizontal, top, bottom, inner and outer indicate orientations or positional relationships based on those shown in the accompanying drawings, which are only for facilitating the description of the present application and simplifying the description, and do not indicate or imply that the referred device or element must have a specific orientation, be constructed and operated in a specific orientation, and thus shall not be construed as limitations on the present application.

In addition, the terms first and second are only used for descriptive purposes, and shall not be construed as indicating or implying relative importance or implicitly indicating the quantity of indicated technical features. Therefore, features defined as first and second may explicitly or implicitly include one or more such features. In the description of the present application, the meaning of a plurality is two or more, unless otherwise clearly and specifically defined.

This application provides a gantry of medical device. The gantry comprises a fixed assembly, a rotating member, and a bearing. The fixed assembly comprises a first base member and a second base member which are detachably connected. The bearing is mounted on the first base member. The rotating member is detachably connected to a rotor of the bearing. The second base member is configured to support the rotating member after the rotating member is separated from the bearing.

The gantry for medical device may include, but is not limited to, a CT (Computed Tomography) gantry, a PET-CT (Positron Emission Tomography-Computed Tomography) gantry, or an MR (Magnetic Resonance) gantry. A CT gantry is used herein as an example to illustrate the technical solutions of the present application.

The CT gantry generally comprises a fixed assembly, a rotating member, and a bearing, wherein the rotating member is mounted on the fixed assembly via the bearing. The fixed assembly primarily serves a supporting role, such as supporting the rotating member. The rotating member carries an X-ray source for emitting an X-ray beam and a detector. After passing through a human body, the X-ray beam is captured by the detector. A computer performs image reconstruction based on the signal captured by the detector to obtain an image of a specific part of the human body. In practical applications, the fixed assembly, the rotating member, and the bearing occupy a large amount of space and are heavy. However, the load-bearing capacity and accommodation space of the transport equipment used for transporting the CT gantry (such as hospital elevators) are limited, resulting in difficulties in the transportation of the CT gantry.

In order to solve the above-mentioned problems, an embodiment of the present application provides a CT gantry with a fixed assembly 100 (also referred to as a fixed gantry) divided into a separable first base member110 and a second base member 120. During transportation of the CT gantry, the rotating member 200 (also referred to as a rotating gantry) can be separated from a bearing 300. The bearing 300 is mounted on the first base member 110, and the rotating member 200 is supported by the second base member 120. This configuration allows the CT gantry to be separated into two parts for individual transportation, thereby reducing the difficulty of transporting the CT gantry.

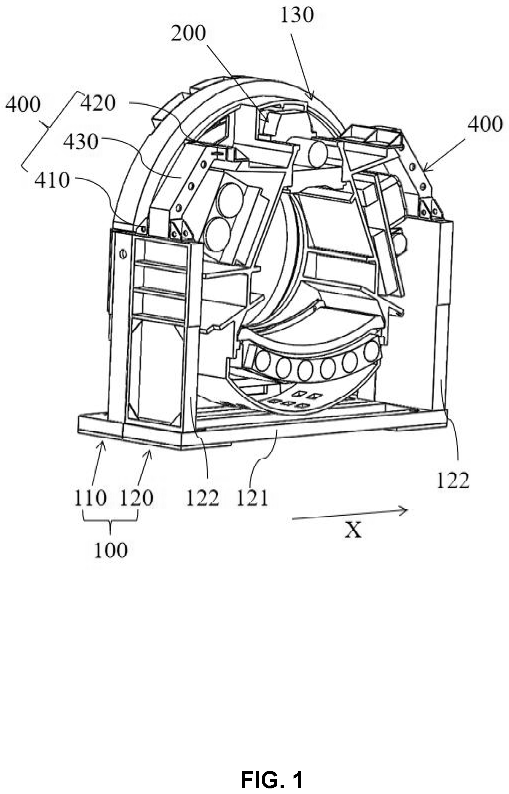

Please refer to FIGS. 1 to 3 for a detailed description of the CT gantry provided in the embodiments of the present application. The CT gantry comprises a fixed assembly 100, a rotating member 200, and a bearing 300. The fixed assembly 100 comprises a first base member 110 and a second base member 120 that are detachably connected. The bearing 300 is mounted on the first base member 110. The rotating member 200 is detachably connected to a rotor 301 of the bearing 300. The second base member 120 is used to support the rotating member 200 after the rotating member 200 is separated from the bearing 300.

The fixed assembly 100 includes a first base member 110 and a second base member 120 that are detachably connected together. The first base member 110 and the second base member 120 can be connected together to form the fixed assembly 100, and the first base member 110 and the second base member 120 can also be separated from each other. The rotating member 200 is constructed to carry an X-ray source and a detector for emitting an X-ray beam. For example, the rotating member 200 has an overall annular structure, and accommodating spaces are formed at corresponding circumferential positions of the rotating member 200 to house the X-ray source and the detector.

The bearing 300 is mounted on the first base member 110. Specifically, a stator 302 of the bearing 300 is mounted on the first base member 110, and the rotating member 200 is connected to a rotor 301 of the bearing 300. When the rotating member 200 is driven to rotate, the rotating member 200 and the rotor 301 of the bearing 300 rotate together relative to the stator of the bearing 300 and the first base member 110. In addition, the rotating member 200 can also be separated from the rotor 301 of the bearing 300.

The second base member 120 is used to support the rotating member 200 after it is separated from the bearing 300. Specifically, when the rotating member 200 is connected to the bearing 300, the rotating member 200 is supported by the bearing 300 and the first base member 110. When the rotating member 200 is separated from the bearing 300, the second base member 120 supports the rotating member 200. In addition, the number of the rotating part 200 can be one or two, the second body 120 is only used to support the rotating part 200 that it is designed to support and which is separated from the bearing 300, whereas the rotating member 200 that is not separated from the bearing 300 continues to be supported by the first base member 110.

After the CT gantry is assembled, the first base member 110 is connected to the second base member 120, with the bearing 300 mounted on the first base member. The rotating member 200 is then connected to the bearing 300, enabling it to rotate on the first base member 110 via the bearing. When the CT gantry is transported, the rotating member 200 is separated from the bearing 300, and the rotating member 200 that is separated from the bearing 300 is supported by the second base member 120, whereas the bearing 300 and the rotating member 200 that is not separated from the bearing 300 remain supported on the first base member 110. The first base member 110 and the second base member 120 are transported separately.

In an embodiment of the present application, the fixed assembly 100 includes a first base member 110 and a second base member 120. The first base member 110 and the second base member 120 are detachably connected. The first base member 110 is used to mount the bearing 300, and the second base member 120 is used to support the rotating member 200 after the rotating member 200 is separated from the bearing 300. Specifically, during transportation, the rotating member 200 can be separated from the bearing 300 and then the rotating member 200 is supported by the second base member 120, thereby allowing the rotating member 200 and the bearing 300 to be transported separately. This reduces the transportation difficulty of the CT gantry and improves its transportation convenience. Meanwhile, by separating the second base member 120 from the fixed assembly 100 to support the separated rotating member 200, the present application avoids the need for an additional support structure to support the separated rotating member 200, thereby reducing the transportation cost and simplifying the transportation assembly process of the fixed assembly 100. In addition, since the rotating member 200 and the bearing 300 are transported separately, damage to the bearing 300 caused by transportation impact on the rotating member 200 can be prevented.

In an embodiment of the present application, referring to FIGS. 1 to 3, the CT gantry comprises a rotating member 200 and a second base member 120. After assembly, the second base member 120 is connected to the first base member 110 to form a fixed assembly 100, and the rotating member 200 is mounted on the bearing 300. During transportation, the rotating member 200 is separated from the bearing 300, the second base member 120 supports the rotating member 200, and the second base member 120 is separated from the first base member 110.

In another embodiment of the present application, the CT gantry comprises two rotating members 200 and two second base members 120. After assembly, the two second base members 120 are connected to opposite sides of the first base member 110 to form a fixed assembly 100. The two rotating members 200 are respectively connected to the bearing 300 and arranged at intervals along an axial direction of the bearing 300, and the two second base members 120 are respectively arranged corresponding to the two rotating members 200. During transportation, the two rotating members 200 are separated from the bearing 300, the two second base members 120 respectively support the corresponding rotating members 200, and the two second base members 120 are separated from the two first base members 110.

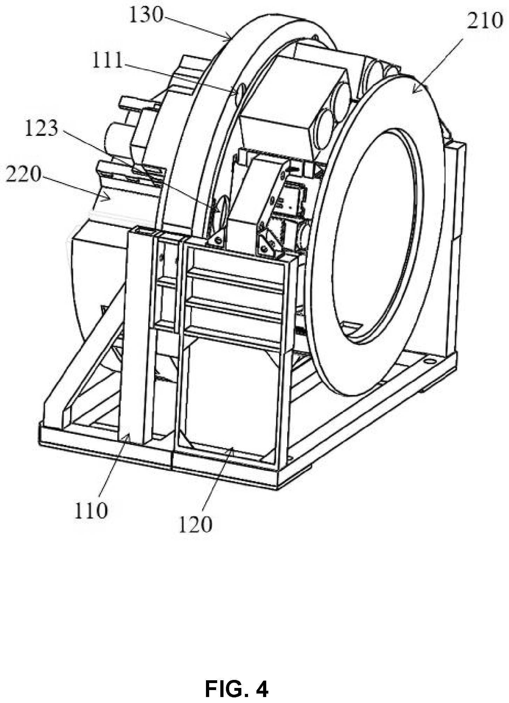

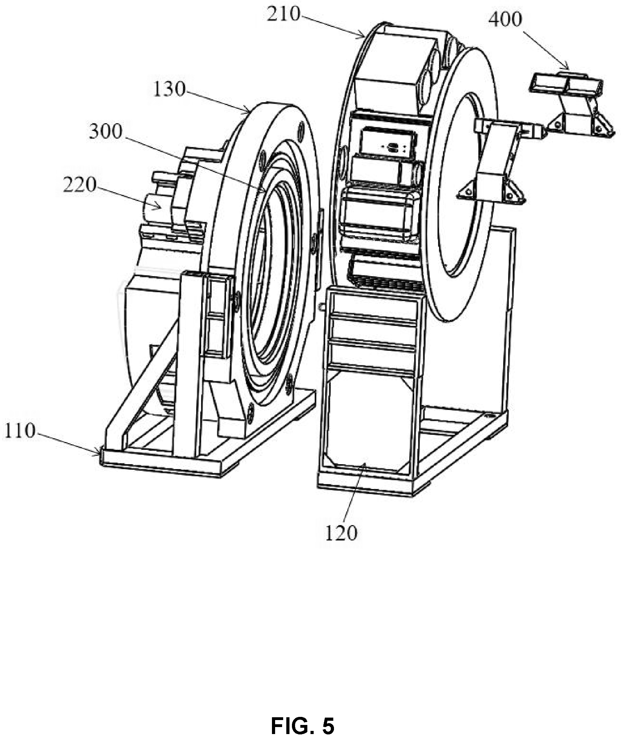

In an embodiment of the present application, referring to FIG.4 and FIG.5, the CT gantry comprises two rotating members 200, respectively, a first rotating member 210 and a second rotating member 220. The first rotating member 210 and the second rotating member 220 are connected to the bearing 300 respectively, and The first rotating member 210 and the second rotating member 220 are arranged spaced apart along an axial direction of the bearing 300. The fixed assembly 100 includes a second base member 120, which is used to support the first rotating member 210 after the first rotating member 210 is separated from the bearing 300.

After assembly, the first rotating member 210 and the second rotating member 220 are respectively connected to the bearing 300, and the first rotating member 210 is mounted on the second base member 120. During transportation, the first rotating member 210 is separated from the bearing 300, the first rotating member 210 is supported by the second base member 120, and the second base member 120 is separated from the first base member 110. The first rotating member 210 is transported together with the second base member 120. The second rotating member 220 remains mounted on the first base member 110 via the bearing 300, and the second rotating member 220 is transported together with the first base member 110. In this embodiment, when the CT gantry comprises two rotating members 200, one of the rotating members 200 and the corresponding second base member 120 can be separated from the bearing 300 for individual transportation. Therefore, it is not necessary to separate both rotating members 200 from the bearings 300, which simplifies the structure of the fixed assembly 100.

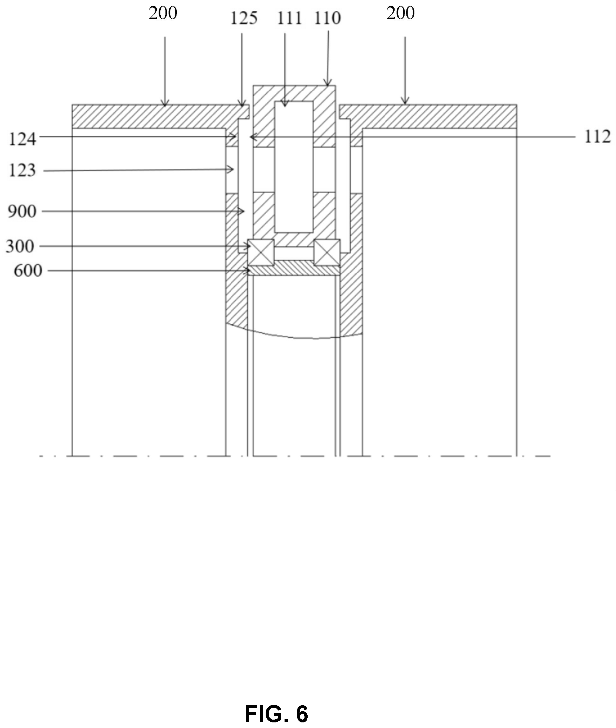

In the aforementioned embodiment pertaining to the two rotating members 200, referring to FIG. 6, the first base member 110 is provided with a synchronizing member 600. The synchronizing member 600 is connected to the rotor 301 of the bearing 300, and both ends of the synchronizing member 600 are respectively connected to the two rotating members 200. By means of the synchronizing member 600 connecting the two rotating members 200, rotational consistency between the two rotating members 200 can be ensured, and the mounting reliability of the rotating members 200 on the first base member 110 can also be improved.

Optionally, referring to FIG. 6, The first base member 110 is provided with two bearings 300. Mounting holes are formed in the first base member 110. The two bearings 300 are mounted in the mounting holes respectively and arranged spaced apart along the axial direction of the mounting holes. The synchronizer 600 is connected to rotors 301 of the two bearings 300, and the two rotating members 200 are connected to opposite ends of the synchronizer 600 respectively. It is understandable that, in other embodiments of the present application, one bearing 300 may also be mounted on the first base member 110; the synchronizer 600 is connected to the rotor 301 of the bearing 300, and the two rotating members 200 are connected to opposite ends of the synchronizer 600 respectively.

In an embodiment of the present application, referring to FIGS. 1 to 3, the CT gantry further includes a connector 400. The connector 400 is used to fix the rotating member 200 on the second base member 120, and the connector 400 is detachably connected to the rotating member 200. When assembling the CT gantry, the connector 400 is separated from the rotating member 200; when transporting the CT gantry, first fix the rotating member 200 on the second base member 120 through the connector 400, then separate the rotating member 200 from the bearing 300, and finally separate the second base member 120 from the first base member 110, so that the second base member 120, the rotating member 200 and the connector 400 are fixed as a whole and can be transported separately.

Herein, the connector 400 may be fixed to the second base member 120, or it may be detachably connected to the second base member 120. When the connector 400 is fixed to the second base member 120, the connector 400 is always disposed together with the second base member 120, and during transportation, it is only necessary to connect the connector 400 to the rotating member 200. When the connector 400 is detachably connected to the second base member 120, the connector 400 is configured to be a separate component during the assembly of the CT gantry. During transportation, the connector 400 is then connected to the second base member 120 and the rotating member 200 respectively, so as to fix the rotating member 200 to the second base member 120.

In an embodiment of the present application, a count of the connectors 400 is at least two, and the at least two connectors 400 are configured to connect the rotating member 200 to the second base member 120 at different positions of the rotating member 200. Herein, the rotating member 200 is installed via the at least two connectors 400, thereby ensuring that the rotating member 200 is stably and securely mounted on the second base member 120.

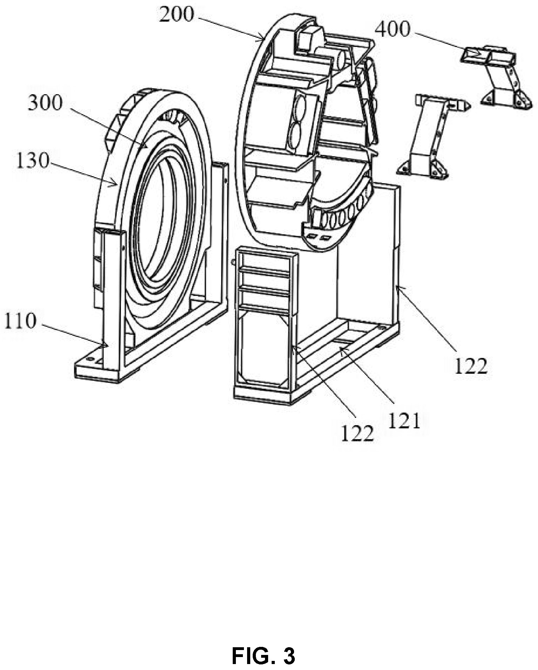

In an embodiment of the present application, referring to FIGS. 1 and 3, the second base member 120 comprises a base plate 121 and two side plates 122. The two side plates 122 are connected to opposite sides of the base plate 121 along a first direction X, and the two connectors 400 are respectively connected between the two side plates 122 and the rotating member 200; specifically, each connector 400 is connected between a top end of the corresponding side plate 122 and the rotating member 200. Herein, the first direction X is perpendicular to an axial direction of and the two connectors 400 are respectively connected between the two side plates 122 and the rotating member 200, and the first direction X is perpendicular to a vertical direction. The second base member 120 in this embodiment is constituted by the base plate 121 and the two side plates 122 arranged spaced apart along the first direction X, thereby making the structure of the second base member 120 simple and enabling the second base member 120 to occupy little space along the axial direction of the rotating member 200. It can be understood that in other embodiments of the present application, the second base member 120 may further comprise a top plate connected to the two side plates 122, wherein the top plate is provided with a connector 400, and the connector 400 is connected between the top plate and the rotating member 200. In addition, in other embodiments, a count of the connectors 400 may also be three, four, or more than four, which is not uniquely limited herein.

In an embodiment of the present application, referring to FIG. 1, the connector 400 comprises a support portion 430, and a first connecting portion 410 and a second connecting portion 420 connected to opposite ends of the support portion 430, wherein the first connecting portion 410 and the second connecting portion 420 are connected to the second base member 120 and the rotating member 200, respectively. Specifically, the first connecting portion 410 is attached to a top face of the side plate 122 and locked/fixed onto the top face of the side plate 122; a mounting surface is formed on and the two connectors 400 are respectively connected between the two side plates 122 and the rotating member 200, and the second connecting portion 420 is attached to the mounting surface and locked/fixed onto the mounting surface. Herein, the provision of the support portion 430 is capable of supporting and suspending the rotating member 200, ensuring that and the two connectors 400 are respectively connected between the two side plates 122 and the rotating member 200 can be suspended and fixed between the two side plates 122 and the base plate 121.

Referring to FIG. 1, the support portion 430 is arranged obliquely from the side plate 122 toward the rotating member 200. Such an arrangement allows the support portion 430 to be positioned closer to the rotating member 200, thereby making the layout of the connector 400, the rotating member 200, and the second base member 120 more compact and reducing the space occupied.

In an embodiment of the present application, the support portion 430, the first connecting portion 410, and the second connecting portion 420 are all sheet metal parts; the support portion 430 is locked to the first connecting portion 410, and the support portion 430 is locked to the second connecting portion 420. It can be understood that, in other embodiments of the present application, the aforementioned support portion 430, first connecting portion 410, and second connecting portion 420 may also all be aluminum profiles, steel, or other metal components, which are not uniquely limited herein.

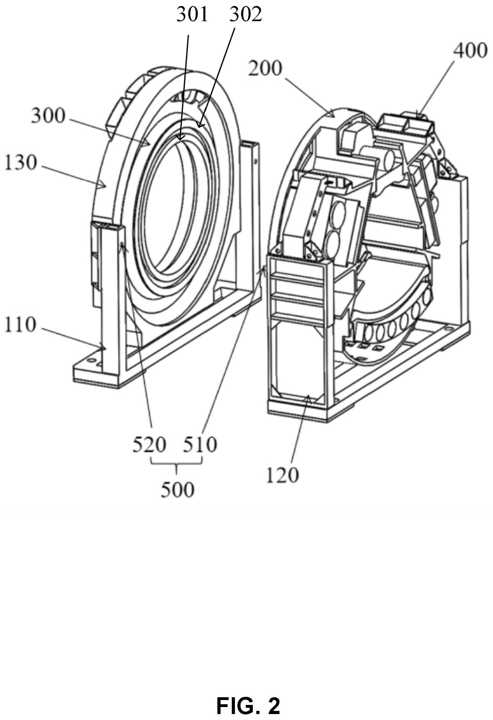

In an embodiment of the present application, referring to FIG. 2, a guidance structure 500 is provided between the first base member 110 and the second base member 120, the guidance structure 500 being configured to guide the assembly of the first base member 110 and the second base member 120 in an axial direction of the bearing 300. Herein, by providing the guidance structure 500, during assembly of the first base member 110 and the second base member 120, it is only necessary to push the first base member 110 and the second base member 120 along the guidance structure 500 to achieve positional engagement between the first base member 110 and the second base member 120, thereby reducing the assembly difficulty and ensuring the assembly accuracy of the first base member 110 and the second base member 120.

In an embodiment of the present application, referring to FIG. 2, the guidance structure 500 comprises a guide groove 520 formed in the first base member 110 and a guide bar 510 formed on the second base member 120, wherein the guide bar 510 is configured to be inserted into and mate with the guide groove 520. During assembly, an end portion of the guide bar 510 can be inserted into the guide groove 520, and then the second base member 120 is pushed until the guide bar 510 is fully inserted into the guide groove 520, thereby achieving accurate positioning between the second base member 120 and the first base member 110.

In a specific configuration, the guide bar 510 is formed on the side of the side plate 122 facing the first base member 110, the guide groove 520 is formed on the side of the first base member 110 facing the second base member 120, and the guide bar 510 extends along the axial direction of the rotating member 200. During assembly, the first base member 110 and the second base member 120 are moved closer to each other along the axial direction of the rotating member 200 so that the guide bar 510 is inserted into the guide groove 520.

Optionally, referring to FIG. 2, the guidance structure 500 comprises two guide bars 510 and two guide grooves 520. The two guide bars 510 are respectively formed on the two side plates 122. The two guide bars 510 and the two guide grooves 520 are provided in a one-to-one correspondence for inserted engagement.

Understandably, in other embodiments of the present application, the guide bar 510 may alternatively be formed on the first base member 110 and the guide groove 520 may be formed on the second base member 120. Furthermore, a count of the guide bars 510 and guide grooves 520 may also be one, three, four, or more than four, and is not uniquely limited herein.

In an embodiment of the present application, a first thread hole is formed on the first body 110 (not shown in the figure), and a second threaded hole is formed in the second base member 120 (not shown in the figures). A locking member is threaded through the first threaded hole and the second threaded hole and locked therein. In the present embodiment, the positioning between the first base member 110 and the second base member 120 is achieved by means of the guidance structure 500, and then the first base member 110 and the second base member 120 are secured by the locking member, thereby ensuring a firm and stable structure of the fixed assembly 100.

Optionally, a count of the first threaded holes and the second threaded holes may also be plural, wherein the first threaded holes are distributed at intervals on the first base member 110, and the second threaded holes are distributed on the second base member 120 with spacing between them. Furthermore, in other embodiments of the present application, the first base member 110 and the second base member 120 may also be connected by means of a snap-fit connection or by means of an interference fit, which is not uniquely limited herein.

In an embodiment of the present application, a first mounting hole is formed in the rotating member 200 (not shown in the figures), and a second mounting hole is formed in the rotor 301 of the bearing 300 (not shown in the figures). A fastener is passed through and locked in the first mounting hole and the second mounting hole respectively, wherein the fastener may be a bolt. By means of the fastener, the rotor 301 of the bearing 300 is locked to the rotating member 200, thereby ensuring the mounting stability of the rotating member 200 on the bearing 300 and avoiding the problem of unstable rotation of the rotating member 200 caused by the rotating member 200 detached from the bearing 300 during rotation.

In an embodiment of the present application, referring to FIGS. 1 to 3, the first base member 110 comprises a bearing support 130. The bearing support 130 is mounted on the first base member 110, and the stator 302 of the bearing 300 is mounted on the bearing support 130. Herein, by providing the bearing support 130, the support stability of the first base member 110 for the bearing 300 can be improved and additionally, the bearing support 130 can be disassembled and maintained when damaged. It can be understood that, in other embodiments of the present application, a support for supporting the bearing 300 may also be integrally formed on the first base member 110, that is, the bearing 300 may be directly mounted on the first base member 110, which is not uniquely limited herein.

In an embodiment of the present application, referring to FIG. 4 and FIG. 6, a first channel 111 is formed on the first base member 110, the first channel 111 is formed on the bearing support 130. A cooler is provided in the first channel 111 and a second channel 123 is formed on the rotating member 200. The second channel 123 is connected with the first channel 111. An airflow generating assembly is disposed on the first base member 110, wherein an air inlet of the airflow generating assembly is in communication with the first channel 111 and an air outlet of the airflow generating assembly is in communication with the second channel 123.

Since the rotating member 200 is equipped with a radioactive source and detector for emitting X-ray beams, as well as some other electronic structures, these electronic structures will generate a large amount of heat after long time operation. When it is necessary to dissipate heat to the rotating member 200, the hot air around the rotating member 200 can be sent to the first channel 111 through the second channel 123 through the airflow generating component. Then the hot air in the first channel 111 can be cooled into cold air through the cooler. Finally, the cold air in the first channel 111 is transported to the periphery of the rotating member 200 through the second channel 123 through the air flow generating assembly. This cycle makes the periphery of the rotating member 200 (each electronic structure is installed on the periphery of the rotating member 200) at the appropriate ambient temperature to avoid the alarm of the device caused by the environmental overtemperature.

In addition, in the present embodiment, the structural space of the first base member 110 itself is utilized to mount the cooler and to accommodate a portion of the cold air. This configuration enables the isolation of cold and hot air, facilitates improved heat dissipation control, and eliminates the need for additional space on the CT gantry to install the cooler.

Optionally, the air flow generating assembly is mounted on the an outer edge of the first base member 110. The airflow generating assembly is arranged opposite the cooler and is configured to deliver air cooled by the cooler into the second channel 123. It can be understood that, in other embodiments of the present application, a plurality of airflow generating assemblies may be distributed on the first base member 110, wherein a first portion of the airflow generating assemblies conveys hot air from the second channel 123 to the first channel 111, and a second portion of the airflow generating assemblies conveys cold air from the first channel 111 to the second channel 123. Furthermore, in practical applications, the CT gantry is enclosed by an outer cover. The outer cover is configured to concentrate hot air from the periphery of the first base member 110, thereby enabling the airflow generating assembly to deliver the hot air into the first channel 111.

The specific structure and working principle of the cooler and the airflow generating assembly will be described in detail in subsequent embodiments.

In an embodiment of the present application, referring to FIG. 6, the first base member 110 has a first end face 112, and the rotating member 200 has a second end face 124. The first end face 112 and the second end face 124 are arranged opposite to and spaced apart from each other. A protruding ring 125 extends from a periphery of the second end face 124 toward the first end face 112, such that the first end face 112, the second end face 124, and the protruding ring 125 together define an isolation chamber 900 therebetween. The isolation chamber 900 is in communication between the first channel 111 and the second channel 123. Herein, the provision of the isolation chamber 900 enables isolation between the first channel 111 and the second channel 123 during operation of the CT gantry, i.e., isolation of the cold and hot air chambers, thereby facilitating improved heat dissipation control by the CT gantry.

According to a second aspect, an embodiment of the present application also provides a heat dissipation method for a CT gantry. The method is applied to the CT gantry comprising the first channel 111, the second channel 123, the airflow generating assembly, and the cooler, and comprises the following steps:
S10: directing, by the airflow generating assembly, hot air from a periphery into the first channel 111 via the second channel 123.

S20: cooling, by the cooler, the hot air in the first channel 111 to form cold air.

S30: delivering, by the airflow generating assembly, the cold air from the first channel 111 to the periphery via the second channel 123.

Herein, the airflow generating assembly in step S10 and the airflow generating assembly in step S30 may be the same device, or may be different airflow generating assemblies. In addition, the airflow generating assembly may be mounted on the first base member 110 or on the second base member 120.

Optionally, the airflow generating assembly may be a fan or a blower.

Optionally, a coolant (cooling medium) circulates within the cooler. Heat exchange between the cooling medium and the high-temperature air stream causes the air stream to be cooled after passing through the cooler, such that the high-temperature air stream becomes a low-temperature air stream, thereby improving the cooling effect. It can be understood that, in other embodiments of the present application, the cooler may also be a refrigeration unit, such as a semiconductor refrigeration unit.

According to a third aspect, an embodiment of the present application also provides a CT device, comprising the CT gantry as described in any of the foregoing embodiments. Furthermore, the CT device also comprises a computer system, an image display system, and a storage system. Herein, an emitter and a receiver on the rotating member 200 form a scanning system of the CT device. The scanning system, the image display system, and the storage system are respectively in communication connection with the computer system. The scanning system is configured to transmit scanning results to the computer system. The computer system is configured to process information based on the scanning signals and stored signals in the storage system, and to display the computed results via the image display system.

It is to be understood that the foregoing embodiments are described merely by way of example using a CT gantry and a CT device. The present application can be applied to various medical devices, such as various medical imaging devices including PET-CT devices and MR devices, or various radiotherapy devices. In these medical devices, the fixed assembly is divided into a first base member and a second base member that are detachably connected. The first base member is used for mounting the bearing, and the second base member is used for supporting the rotating part after it is detached from the bearing. During transportation, the rotating part can be detached from the bearing and then supported by the second base member, thereby enabling the rotating part and the bearing to be transported separately. This reduces the transportation difficulty and improves the transportation convenience of the gantry.

Heating elements, such as the radiation source and the detector of the CT device, generate a significant amount of heat during operation. Excessively high temperatures can seriously affect the performance of the CT device. Therefore, an effective cooling system is crucial for the normal operation of the CT device, and air cooling is currently the primary cooling method.

In related art, to achieve a better cooling effect, multiple sets of airflow generating assemblies are often arranged within the rotating gantry. These multiple sets of airflow generating assemblies operate simultaneously; however, the airflow cannot form a stable path, resulting in high noise levels from the CT device.

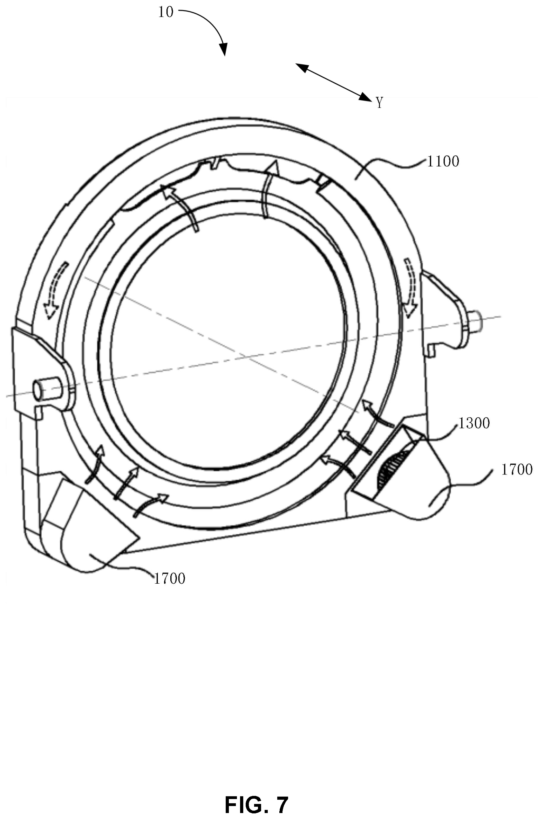

Referring to FIGS. 7 to 9, an embodiment of the present application provides a gantry 10 for a medical imaging device. The gantry 10 comprises a fixed gantry 1100, a rotating gantry 1200, and an airflow generating assembly 1300. The fixed gantry 1100 primarily serves a supporting function, for example, by supporting the rotating gantry 1200, and its structure can be as described in the foregoing embodiments. The fixed gantry 1100 may be an integral structure or a split structure as described in the previous embodiments. The following embodiments are described using the integral structure as an example. However, it is to be understood that the structural features of the fixed gantry 1100 described below are also applicable to the split structure. The rotating gantry 1200 is configured to carry a radiation source and a detector for emitting an X-ray beam. For example, the rotating member 200 is configured as an annular structure, and an accommodation space is formed at a corresponding circumferential portion of the rotating member 200 to accommodate the radiation source and the detector.

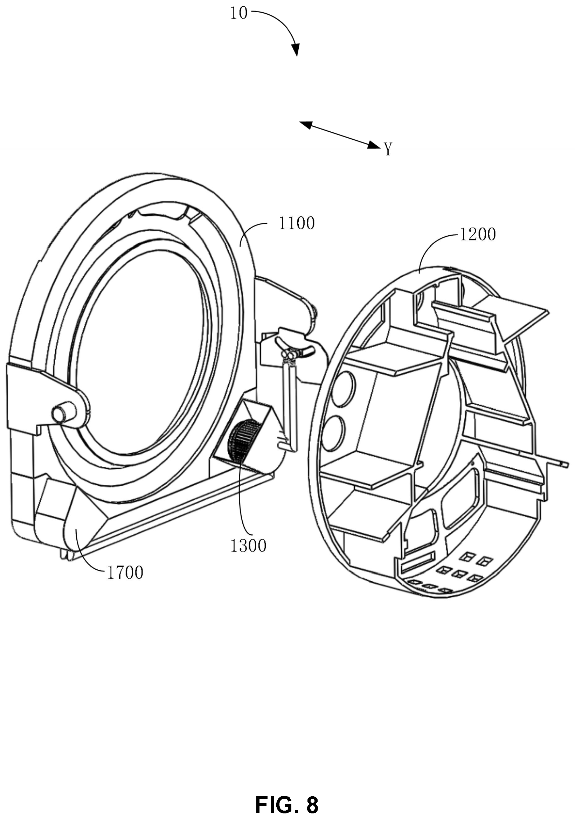

A first channel 1110 is formed inside the fixed gantry 1100. Herein, the first channel 110 may be an annular structure arranged along a circumferential direction of the fixed gantry 1100, meaning that the first channel 1110 is connected end to end, or the first channel 1110 may be of a segmented type, meaning that a plurality of first channels 1110 arranged spaced apart along the circumferential direction of the fixed gantry 1100. The rotating gantry 1200 is rotatably connected to an axial side of the fixed gantry 1100, where the axial direction is indicated by an arrow Y in FIG. 8. A second channel 1210 is formed inside the rotating gantry 1200. The second channel 1210 may be an annular structure or a segmented structure are spaced apart along the circumferential direction. The airflow generating assembly 1300 is connected to the fixed gantry 1100. An air inlet of the airflow generating assembly 1300 is in communication with the first channel 1110, and an air outlet of the airflow generating assembly 1300 is in communication with the second channel 1210. The first channel 1110, the airflow generating assembly 1300, and the second channel 1210 collectively define a passage for circulating a gas. It can be understood that the fluid communication between the air inlet and the first channel 1110 may mean that the two are in direct contact so as to be in a connected state; or there may be a gap between the air inlet and the first channel 1110, wherein the gap is capable of allowing an airflow to pass therethrough so that the airflow can flow from the first channel 1110 to the air inlet; or even that a connecting pipeline is provided between the air inlet and the first channel 1110 so that the airflow can flow between the two.

Thus, when heat generated by a heating element inside the rotating gantry 1200 during operation is transferred to the fixed gantry 1100, the high-temperature air flow within the first channel 1110 can be guided to the airflow generating assembly 1300 due to the fluid communication between the first channel 1110 and the air inlet of the airflow generating assembly 1300; after being cooled by the airflow generating assembly 1300, it is then guided into the second channel 1210 of the rotating gantry 1200.

Taking as an example that the first channel 1110 is annular and a plurality of second channels 1210 are provided: since the first channel 1110 is annular and the plurality of second channels 1210 are arranged along the circumference, the gas cooled by the airflow generating assembly 1300 can enter the rotating gantry 1200 through the plurality second channels 1210 at different positions, thereby dissipating heat from and cooling the heating elements inside the rotating gantry 1200.

It is not necessary to arrange multiple sets of airflow generating assemblies 1300; only one airflow generating assembly 1300 needs to be provided. By configuring the annular first channel 1110 in cooperation with the multiple second channels 1210, the cooling air flow enters the rotating gantry 1200 at different positions, enabling effective cooling at multiple circumferential locations of the rotating gantry 1200. This reduces the space occupied by circumferentially arranging multiple sets of airflow generating assemblies 1300, resulting in a medical imaging device that occupies less space and has lower manufacturing costs; it also reduces the additional noise generated by the high-speed operation of multiple sets of airflow generating assemblies 1300, thereby improving patient comfort.

Herein, the airflow generating assembly 1300 may be a fan or the like. In order to ensure the operational stability of the fan, the gantry 10 is further provided with a mounting housing 1700, and the fan is mounted on the mounting housing 1700.

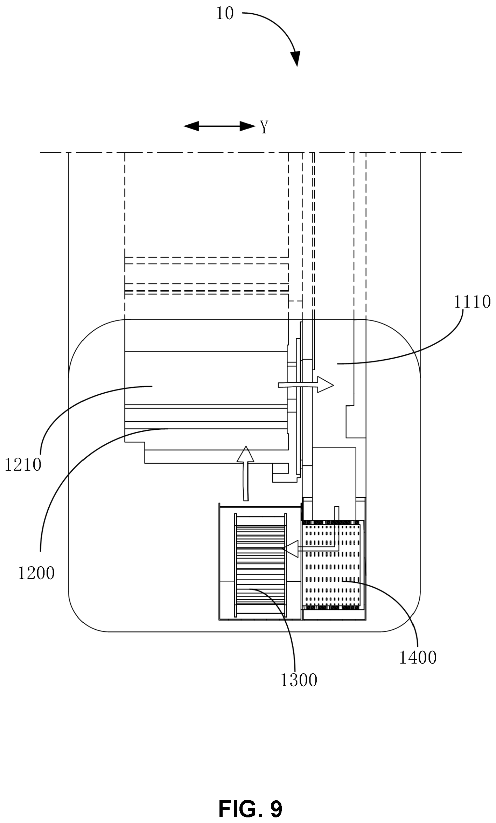

In an embodiment of the present application, referring to FIG. 9, an air inlet of the airflow generating assembly 1300 faces the first channel 1110 and an air outlet of the airflow generating assembly 1300 faces the rotating gantry 1200. This configuration allows high-temperature air flow in the first channel 1110 to rapidly enter the airflow generating assembly 1300, and allows low-temperature air flow after heat exchange with the airflow generating assembly 1300 to rapidly enter the rotating gantry 1200. That is, the flow path of the air flow is shorter, resulting in lower energy loss and higher cooling efficiency.

In an embodiment of the present application, referring to FIGS. 7 to 9, the first channel 1110 comprises at least one annular channel, meaning that the first channel 1110 is connected end to end. When a count of the annular channels is one, the annular channel may be arranged along a circumferential direction of the fixed gantry 1100. In other embodiments, the first channel 1110 may also be arranged in a spiral pattern along the circumferential direction of the fixed gantry 1100.

In an embodiment of the present application, when the number of the annular channels is plural, each annular channel is arranged along a circumferential direction of the fixed gantry 1100, and the plurality of annular channels may be arranged at intervals along a radial direction of the fixed gantry 1100, that is, the plurality of annular channels are arranged in the form of concentric rings.

In an embodiment of the present application, the first channel 1110 comprises at least one segmental channel, wherein the segmental channel is arranged along a circumferential direction of the fixed gantry 1100. When the number of the segmental channels is plural, the plurality of segmental channels may be arranged along the circumferential direction of the fixed gantry 1100, that is, the plurality of segmental channels are arranged on the same virtual ring. In other embodiments, the plurality of segmental channels may be arranged along a radial direction of the fixed gantry 1100, that is, each segmental channel is located on a virtual ring having a different radial dimension.

In an embodiment of the present application, the arrangement of the second channel 1210 may be implemented by referring to that of the first channel 1110. Specifically, whether annular channels or segmental channels are adopted, and/or whether the number is one or more, may be configured according to actual requirements and will not be described redundantly herein.

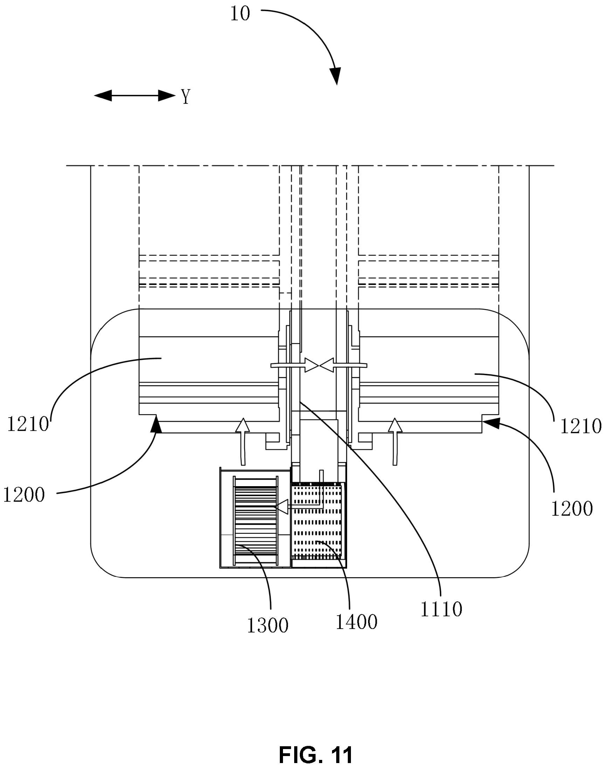

Referring to FIG. 11, in another embodiment, the gantry 10 may comprise two rotating gantries 1200. Correspondingly, the two rotating gantries 1200 are symmetrically connected to opposite sides of the fixed gantry 1100 along the axial direction. Since a scanning mechanism is disposed on each rotating gantry 1200, by providing the two rotating gantries 1200, the scanning resolution can be improved according to actual needs. Herein, the first channel 1110 extends through the fixed gantry 1100 in the axial direction, such that the first channel 1110 can be in communication with the second channels 1210 in the two rotating gantries 1200.

In an embodiment of the present application, there is at least one first channel 1110, and the at least one first channel 1110 is arranged on one side of the fixed gantry1100, that is, the at least one first channel 1110 does not extend through the fixed gantry 1100 in the axial direction, wherein the second channels 1210 of both rotating gantries 1200 are in communication with the first channel 1110. When the at least one first channel 1110 is provided, for example, the at least one first channel 1110 is located on a left side, and a left end of the at least one first channel 1110 is open, thereby enabling direct fluid communication with the second channel 1210 on the left side. Fluid communication between the first channel 1110 and the second channel 1210 of the right rotating gantry 1200 can be achieved via a conduit. When a plurality of first channels are provided, the plurality of first channels are also located on the same side of the fixed gantry 1100. The second channel 1210 on the same side can be in direct fluid communication with the first channels 1110, and the second channel 1210 on the opposite side can be in indirect fluid communication via a connecting pipe 1510.

In an embodiment of the present application, there are at least two first channels. The at least two first channels are respectively arranged on opposite sides of the fixed gantry 1100, meaning that the at least two first channels do not extend through to each other. The second channels 1210 of the two rotating gantries 1200 are respectively in direct or indirect fluid communication with the first channel 1110 located on the same side. This configuration shortens the flow path of the air flow between the first channel 1110 and the second channel 1210 on the same side, thereby allowing the low-temperature air flow to rapidly enter the rotating gantry 1200.

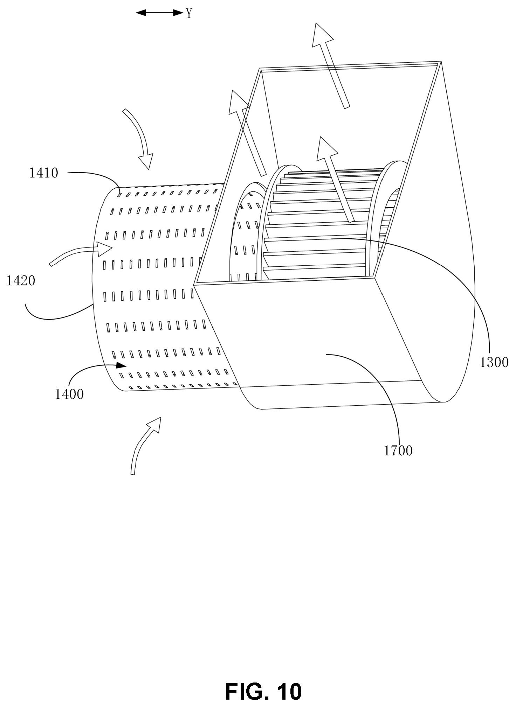

In an embodiment of the present application, referring to FIG. 9 and FIG.10, the gantry 10 further comprises a cooler 1400 connected between the fixed gantry 1100 and the airflow generating assembly 1300. The cooler 1400 is configured to perform heat exchange with a gas flowing out of the first channel 1110. Through the cooperation of the cooler 1400 and the airflow generating assembly 1300, high-temperature gas flowing out of the first channel 1110 is rapidly cooled, thereby cooling the rotating gantry 1200. Furthermore, a coolant circulates within the cooler 1400. Heat exchange between the coolant and the high-temperature air stream causes the air stream to be cooled after passing through the cooler 1400, such that the high-temperature air stream becomes a low-temperature air stream, thereby improving the cooling effect. Herein, the cooler 1400 may be connected to the aforementioned mounting housing 1700.

In an embodiment of the present application, referring to FIG. 9, the first channel 1110 is annular, and the cooler 1400 is mounted within the first channel 1110. This arrangement, on one hand, reduces the additional space occupied by the cooler 1400 and, on the other hand, shortens the flow path of the air stream, thereby allowing the high-temperature air stream to be cooled rapidly. In other embodiments, the cooler 1400 is connected to an exterior of the fixed gantry 1100, wherein an air inlet of the cooler 1400 is in communication with the first channel 1110, and an air outlet of the cooler 1400 is in communication with an air inlet of the fan.

In an embodiment of the present application, referring to FIG. 9 and FIG.10, the cooler 1400 comprises a gas channel communication with the first channel 1110, such that a high-temperature air stream exiting the first channel 1110 is able to flow into the gas channel. The cooler 1400 further includes a fluid channel 1420 configured to allow a coolant to flow through the fluid channel 1420, and perform heat exchange with the air stream in the gas channel. The heat exchange causes the air stream to be cooled as it passes through the cooler 1400, thereby converting the high-temperature air stream into a low-temperature air stream and improving the cooling effect. The coolant may be cooling water or the like.

In an embodiment of the present application, referring to FIG. 9 and FIG.10, the gas channel comprises a plurality of ventilation holes 1410 arranged at intervals along a circumference of the cooler 1400. Each ventilation hole 1410 is in communication with the first channel 1110. Correspondingly, the fluid channel 1420 is a hollow cavity formed inside the cooler 1400. The hollow cavity is in communication with each ventilation hole 1410, such that the high-temperature air stream flowing from the first channel 1110 can enter the internal hollow cavity via the ventilation holes 1410 disposed around the cooler 1400, thereby achieving heat exchange to cool the high-temperature air stream.

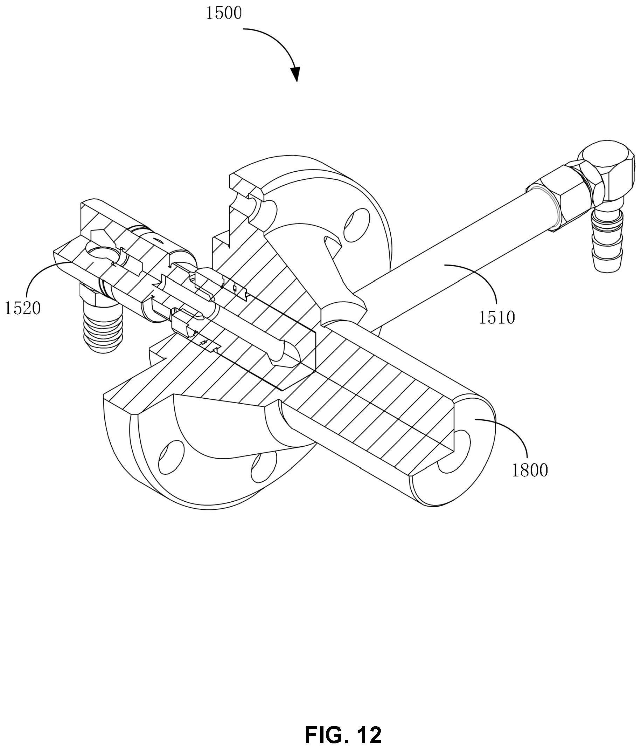

In an embodiment of the present application, referring to FIG. 12, the gantry 10 further comprises a connector assembly 1500 movably connected to the fixed gantry 1100. The connector assembly 1500 comprises a connecting pipe 1510 and a rotary joint 1520 connected to the connecting pipe 1510. The connecting pipe 1510 is configured to be connected to a coolant unit. The rotary joint 1520 is configured to be in fluid communication with the fluid channel of the cooler 1400, so as to introduce a coolant into or discharge the coolant from the fluid channel 1420, thereby ensuring the heat exchange effect between the coolant and the high-temperature air stream. By means of rotation of the rotary joint 1520, it adapts to attitude adjustments of the gantry 10, such as a tilting operation of the gantry 10. The rotary joint 1520 may, for example, be threaded onto the connecting pipe 1510 to achieve relative rotation of the rotary joint 1520 with respect to the connecting pipe 1510.

Furthermore, two sets of the connector assembly 1500 are provided; that is, two sets of the connecting pipe 1510 and the rotary joint 1520 are provided. One set of rotary joints 1520 is in communication with an inlet of the fluid channel 1420 of the cooler 1400 via one set of connecting pipes 1510, so as to introduce a low-temperature cooling medium into the cooler 1400 to cool the air stream. The other set of rotary joints 1520 is in communication with an outlet of the fluid channel 1420 via the other set of connecting pipes 1510. After the heat exchange is completed, the coolant with an elevated temperature is discharged via the other connector assembly 1500, thereby ensuring the supply of the coolant.

As shown in FIG. 12, in some embodiments, a connection base 1800 is further provided on the connector assembly 1500. The connection base 1800 has a hollow structure, meaning a cavity is formed inside it. The rotary joint 1520 is threadedly connected within the cavity, and the connecting pipe 1510 is in communication with the cavity through a slot provided on the connection base 1800. The coolant flows into the cooler 1400 sequentially through the connecting pipe 1510, the cavity, and the rotary joint 1520.

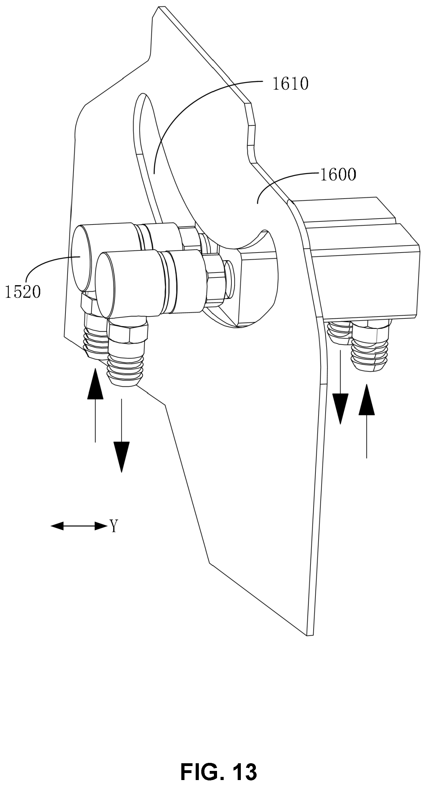

In an embodiment of the present application, the gantry 10 is connected to a deflection shaft, enabling the gantry 10 to tilt about the deflection shaft. By tilting the gantry 10, the scanning position is altered, enabling effective scanning in special postures and obviating the need for frequent adjustments of the patient's position by the physician. For instance, when examining areas such as the cervical or lumbar vertebrae where observation of the intervertebral disc is required, and since the intervertebral space possesses a certain inclination angle, tilting the gantry10 to scan parallel to the lumbar isthmus during lumbar spine scanning allows clear visualization of the width of the lumbar isthmus fissure and any joint disorders. Consequently, a complete image of the intervertebral disc can be obtained. As shown in FIG. 13, a partition plate 1600 is disposed on the gantry 10. A sliding slot 1610 is formed in the partition plate 1600. The connector assembly 1500 is configured to be operably slidable within the sliding slot 1610. In this manner, when the gantry 10 undergoes tilting deflection, the connector assembly 1500 adaptively slides within the sliding slot 1610, thereby accommodating its positional change.

Furthermore, an embodiment of the present application also provides a medical imaging device (not shown in the figures), comprising a radiation source, a detector, and the gantry 10 according to any of the foregoing embodiments. The radiation source and the detector are both mounted on the rotating gantry. The radiation source is configured to emit X-rays, which are captured by the detector after passing through a human body, ultimately generating a digital image. Since this medical imaging device comprises the gantry 10 according to any of the aforementioned embodiments, it is unnecessary to arrange multiple sets of airflow generating assemblies. Only one airflow generating assembly needs to be provided. Through the cooperation of the first channel and the second channel, multiple circumferential positions of the rotating gantry can be effectively cooled. This reduces the space occupied by circumferentially arranging multiple sets of airflow generating assemblies, resulting in a medical imaging device that occupies less space and has lower manufacturing costs. Simultaneously, it reduces the additional noise generated by the high-speed operation of multiple sets of airflow generating assemblies, thereby improving patient comfort.

It is understandable that the medical imaging apparatus further comprises an examination table, and a subject to be examined is placed on the examination table. A cylindrical opening is formed in the middle part of the gantry 10 to allow the examination table to pass therethrough, so as to perform scanning operations on a patient. The gantry 10 can be tilted in a predetermined direction or rotated by a predetermined angle. The examined subject may be organs or blood vessels such as liver, heart, uterus, brain and abdomen. In addition, the subject may further include a human body phantom. The human body phantom refers to a specially designed object having volume, density and effective atomic number close to those of living organisms, and may include a spherical human body phantom with characteristics similar to those of the human body.

It can be appreciated that the present application can also be applied to other medical devices, such as devices for performing radiotherapy on human bodies. The above embodiments are described merely by taking medical imaging apparatuses as examples, and the present application is also applicable to other medical devices including devices for human body radiotherapy. Therefore, the present application is intended to protect all medical devices defined by the appended claims. In actual application scenarios, the medical imaging apparatus may be a CT apparatus and the like.

All technical features of the above embodiments can be combined arbitrarily. For the sake of concise description, all possible combinations of the technical features in the above embodiments are not elaborated herein. Nevertheless, any combination of such technical features without conflicts shall be deemed to fall within the scope recorded in the present specification.

The above descriptions are merely preferred embodiments of the present application, and are not intended to limit the present application. Any modification, equivalent replacement and improvement made within the spirit and principle of the present application shall all fall within the protection scope of the present application.

## Claims

1. A gantry for a medical device, comprising:
a fixed assembly (100) including a first base member (110) and a second base member (120) , wherein the first base member (110) and the second base member (120) are detachably connected to each other;
a bearing (300) mounted on the first base member (110); and
a rotating member (200) detachably connected to a rotor (301) of the bearing (300), wherein the second base member (120) is configured to support the rotating member (200) when the rotating member (200) is detached from the bearing (300).

2. The gantry for the medical device of claim 1, wherein the gantry for medical device also comprises a connector (400), the connector (400) is configured to secure the rotating member (200) to the second base member (120), and the connector (400) is detachably connected to the rotating member (200).

3. The gantry for the medical device of claim 2, wherein a count of the connector is at least two, and the at least two connectors (400) are configured to connect the rotating member (200) to the second base member (120) at different positions on the rotating member (200).

4. The gantry for the medical device of claims 1-3, wherein a guidance structure (500) is provided between the first base member (110) and the second base member (120), and the guidance structure (500) is configured to guide the first base member (110) and the second base member (120) in an axial direction of the bearing (300) during assembly.

5. The gantry for the medical device of claims 1-4, wherein the gantry for the medical device comprises two rotating members (210, 220), the two rotating members (210, 220) includes a first rotating member (210) and a second rotating member (220), each of the first rotating member (210) and the second rotating member (220) is connected to the bearing (300) respectively;
wherein the second base member (120) is configured to support the first rotating member (210) after the first rotating member (210) is detached from the bearing (300), and wherein the second rotating member (220) is mounted on the first base member (110) through the bearing (300) after the first rotating member (210) is detached from the bearing (300).

6. The gantry for the medical device of claim 5, wherein a synchronizer (600) is disposed on the first base member (110), the synchronizer (600) being connected to the rotor (301) of the bearing (300), and the synchronizer (600) having opposite ends connected to the first rotating member (210) and the second rotating member (220), respectively.

7. The gantry for the medical device of claims 1-6, wherein the first base member (110) has a first channel (111), and the rotating member (200) has a second channel (123), the second channel (123) being in communication with the first channel (111); and wherein an airflow generating assembly (1300) is disposed on the first base member (110), an air inlet of the airflow generating assembly (1300) is in communication with the first channel (111) and an air outlet of the airflow generating assembly (1300) is in communication with the second channel (123).

8. The gantry for the medical device of claim 7, wherein a cooler (1400) is provided within the first channel (111) and the cooler (1400) is configured to perform heat exchange with gas flowing out of the first channel (111).

9. The gantry for the medical device of claim 7, wherein the first base member (110) has a first end face (112) and the rotating member (200) has a second end face (124), the first end face (112) and the second end face (124) are opposite and spaced apart from each other, a protruding ring (125) extends from a periphery of the second end face (124) towards the first end face (112), such that the first end face (112), the second end face (124), and the protruding ring (125) together define an isolation chamber (900), and the isolation chamber (900) is in communication between the first channel (111) and the second channel (123).

10. The gantry for medical device of claim 1-9, wherein the gantry for the medical device includes a computed tomography (CT) gantry.

11. A medical device, comprising the gantry for the medical device according to any one of claims 1 to 10.

12. A gantry (10) for a medical device, comprising:
a fixed gantry (1100), internally provided with a first channel (1110);
a rotating gantry (1200), rotatably connected to the fixed gantry (1100), wherein the rotating gantry (1200) is internally provided with a second channel (1210);
an airflow generating assembly (1300), connected to the fixed gantry (1100), wherein an air inlet of the airflow generating assembly (1300) is in communication with the fixed gantry (1100), and an air outlet of the airflow generating assembly (1300) is in communication with the second channel (1210); and
wherein the fixed gantry (1100), the airflow generating assembly (1300), and the second channel (1210) collectively define a passage for circulating gas.

13. The gantry (10) for the medical device of claim 12, wherein the air inlet of the airflow generating assembly (1300) faces the first channel (1110), and the air outlet of the airflow generating assembly (1300) faces the rotating gantry (1200).

14. The gantry (10) for the medical device of claim 12 or claim 13, wherein the gantry (10) for the medical device comprises a cooler (1400) connected between the fixed gantry (1100) and the airflow generating assembly (1300); wherein the cooler (1400) is configured to perform heat exchange with gas flowing out of the first channel (1110).

15. The gantry (10) for the medical device of claim 14, wherein the cooler (1400) is installed within the first channel (1110); the cooler (1400) comprises a gas channel and a fluid channel, the gas channel is in communication with the first channel (1110), and the fluid channel is configured to allow a coolant to flow so as to perform heat exchange with gas in the gas channel.

16. The gantry (10) for the medical device of claim 15, wherein the gas channel comprises a plurality of ventilation holes (1410) arranged spaced apart along a circumference of the cooler (1400), and wherein each of the plurality of ventilation holes (1410) is in communication with the first channel (1110).

17. The gantry (10) for the medical device of claim 15 or claim 16, wherein the gantry for the medical device comprises a connector assembly (1500) movably connected to the fixed gantry (1100), wherein:
the connector assembly (1500) comprises a connecting pipe (1510) and a rotary joint (1520) connected to the connecting pipe (1510);
the connecting pipe (1510) is configured to be connected to a coolant unit; and the rotary joint (1520) is configured to be in communication with the fluid channel of the cooler (1400) so as to introduce a coolant into the the fluid channel or discharge the coolant from the fluid channel.

18. The gantry (10) for the medical device of claim 17, wherein a sliding slot (1610) is provided on the fixed gantry (1100), and the connector assembly (1500) is configured to be operably slidable within the sliding slot (1610).

19. The gantry (10) for the medical device of any one of claims 12 to 18, wherein the first channel (1110) comprises at least one segmental channel, the at least one segmental channel is arranged along a circumferential direction and/or a radial direction of the fixed gantry (1100), and/or wherein the second channel (1210) comprises at least one annular channel, the at least one annular channel is arranged along a circumferential direction and/or a radial direction of the rotating gantry (1200), or wherein the second channel (1210) comprises at least one segmental channel, the at least one segmental channel is arranged along a circumferential direction and/or a radial direction of the rotating gantry (1200).

20. A medical device, comprising the gantry (10) for the medical device of any one of claims 12 to 19.
